# EUROPEAN PATENT APPLICATION

(11) **EP 4 714 944 A1**
(43) Date of publication of application: **25.03.2026**
(21) Application number: 25204161.1
(22) Date of filing: 23.09.2025
(51) Int. Cl.: C07D 213/72

(54) **PROCESS FOR THE PRODUCTION OF 4-AMINO-5-METHYL-(1H)-PYRIDIN-2-ONE AND DERIVATIVES**

(30) Priority: 23.09.2024 EP 24202048
(71) Applicant: MinAscent Technologies GmbH, 06237 Leuna (DE)
(72) Inventor: KÜHNAST, Martin, 06114 HALLE (SAALE) (DE); NONNENMACHER, Jean, 06217 MERSEBURG (DE); RICHTER, Yvonne, 06249 MÜCHELN (GEISELTAL) (DE)
(74) Representative: Plasseraud IP

(57) **Abstract**

The invention relates to an improved process for the production of 4-amino-5-methyl-(1H)-pyridin-2-one and derivatives, wherein 4-hydroxy-5-methyl-(1H)-pyridin-2-one is reacted under pressure with an ammonium salt without adding aqueous ammonia or feeding in gaseous ammonia to provide the target compounds in high yield and high purity.

## Description

### Technical Field

The instant application concerns an improved process for the production of 4-amino-5-methyl-(1H)-pyridin-2-one (formula IV) and derivatives:

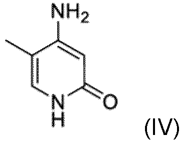

### Background and prior art

The compound of formula (IV) is a key intermediate for the synthesis of finerenone:

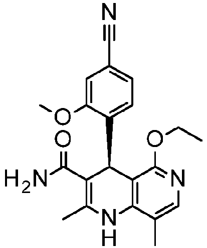

Finerenone has efficacy as non-steroidal mineralocorticoid receptor antagonist (MRA). It is used as a medicament for the prophylaxis and/or treatment of cardiovascular or renal diseases.

The compound of formula (IV) is also an intermediate for the synthesis of omeprazole-derivatives as disclosed in CN103193704 A.

A synthesis starting from the hydroxy-substituted isomer of compound (IV) and affording compound (IV) in one step is described in WO 2020/178177 A1 (Bayer AG). The publication discloses that an amino group can be introduced by reaction with ammonia under addition of an ammonium bromide salt in an autoclave (high pressure reactor) thus affording compound (IV) at a yield of more than 90%. It is disclosed that 0.2 to 3 equivalents, preferably one equivalent, of an ammonium bromide, such as ammonium bromide or tri-alkyl-ammonium bromide or a tetra-alkyl-ammonium bromide, preferably ammonium bromide, are reacted in an autoclave, into which ammonia is fed by condensation to function as reagent and solvent (40 to 100 equivalents, preferably 40 to 60 equivalents, with examples having 50 equivalents of ammonia ). WO 2020/178177 A1 discloses reaction temperatures of 150 to 200°C, and the pressure in the autoclave goes up to 70 to 90 bar as per examples.

The process disclosed in WO 2020/178177 A1 suffers from the disadvantage that large amounts of ammonia are necessary due to its use as solvent. This requires special equipment to conduct the reaction, for safe removal of ammonia after completion of the reaction as well as for recycling of ammonia. Also, after removal of ammonia a dry powder is obtained, which is more difficult to handle and discharge from a large reactor than a solution or slurry.

Therefore, there is a need to provide an alternative synthesis of 4-amino-5-methyl-(1H)-pyridin-2-one (formula IV) and derivatives, which is suitable for industrial production, avoids excessive use of solvents and reagents, in particular ammonia, and can be carried out with standard equipment.

### Summary

The present disclosure provides a process for the production of a 4-amino-(1H)-pyridin-2-one of the following formula (II) 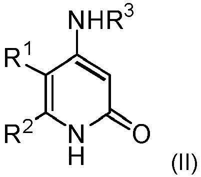 characterised in that a 4-hydroxy-(1H)-pyridin-2-one of the following formula (I) 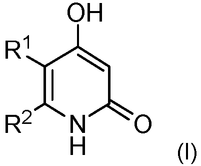 is reacted under pressure with a salt comprising an ammonium cation and in the absence of ammonia (NH₃).

In particular, the present disclosure provides a process for the production of 4-amino-5-methyl-(1H)-pyridin-2-one of formula (IV) 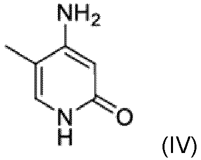 characterised in that 4-hydroxy-5-methyl-(1H)-pyridin-2-one of formula (III) 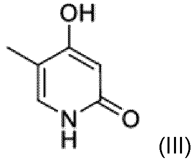 is reacted under pressure with a salt comprising an ammonium cation and in the absence of ammonia (NH₃).

The process of the present disclosure overcomes the above disadvantages of the prior art. Advantages of the process are that the reaction mixture is not or much less corrosive to enamel coatings of reaction container surfaces, since gaseous ammonia as reagent is not used. Therefore special, costly coatings of reactors are not necessary. Glass lining may be used.

Also, special equipment for the handling of vast amounts of gaseous ammonia, its removal after the reaction, and its recycling are not required.

The reaction can be run in standard solvents such as water, which is environmentally friendly.

The reaction may be run at a much lower pressure (below 10 bar) than processes of the prior art. Therefore, the reaction can be run in simpler and less costly reactors.

Vast volumes of solvents are neither required for the reaction, nor for the work-up of the crude product. The work-up of the reaction is easier to carry out using standard equipment to discharge a slurry from the reactor followed by the filtration of the slurry. The process of the present disclosure affords products in high to very high yields.

### Detailed Description

Further aspects, features and advantages of the exemplary embodiments will become apparent from the detailed description which follows.

The patents, published applications and scientific literature referred to herein establish the knowledge of those with skill in the art and are hereby incorporated by reference in their entireties to the same extent as if each was specifically and individually indicated to be incorporated by reference.

As used herein, whether in a transitional phrase or in the body of a claim, the terms "comprise(s)" and "comprising" are to be interpreted as having an open-ended meaning. That is, the terms are to be interpreted synonymously with the phrases "having at least" or "including at least". When used in the context of a method, the term "comprising" means that the method includes at least the recited steps but may include additional steps.

The terms "consists essentially of" or "consisting essentially of" have a partially closed meaning, that is, they do not permit inclusion of steps or features or components which would substantially change the essential characteristics of a method or composition; for example, steps or features or components which would significantly interfere with the desired properties of the compounds or compositions described herein, i.e., the method or composition is limited to the specified steps or materials and those which do not materially affect the basic and novel characteristics of the method or composition. The terms "consists of" and "consists" are closed terminology and allow only for the inclusion of the recited steps or features or components.

As used herein, the singular forms "a," "an" and "the" specifically also encompass the plural forms of the terms to which they refer, unless the content clearly dictates otherwise.

The term "about" is used herein to mean approximately, in the region of, roughly, or around. When the term "about" is used in conjunction with a numerical range, it modifies that range by extending the boundaries above and below the numerical values set forth.

The term "dissolved" or "substantially dissolved" is used herein to mean the solubilization of a solid in a solution. It can be considered that a solid is "dissolved" or "substantially dissolved" in a solution when the resulting solution is clear or substantially clear.

As used herein, the recitation of a numerical range for a variable is intended to convey that the variable can be equal to any values within that range. Thus, for a variable which is inherently discrete, the variable can be equal to any integer value of the numerical range, including the end-points of the range. Similarly, for a variable which is inherently continuous, the variable can be equal to any real value of the numerical range, including the end-points of the range. As an example, a variable which is described as having values between 0 and

2, can be 0, 1 or 2 for variables which are inherently discrete, and can be 0.0, 0.1, 0.01, 0.001, or any other real value for variables which are inherently continuous.

In the specification and claims, the singular forms include plural referents unless the context clearly dictates otherwise.

Technical and scientific terms used herein have the meaning commonly understood by one of skill in the art to which the present description pertains, unless otherwise defined.

The process of the present disclosure is characterized in that a 4-hydroxy-(1H)-pyridin-2-one of the following formula (I) 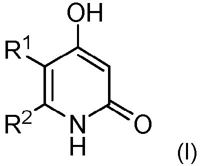 is reacted under pressure with a salt comprising an ammonium cation and in the absence of ammonia (NH₃) to afford a 4-amino-(1H)-pyridin-2-one of the following formula (II)

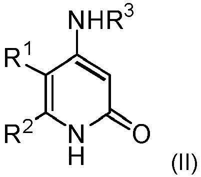

In formulas (I) and (II) substituents R¹ and R² may be independently selected from linear or branched alkyl groups, aryl, benzyl, or hydrogen. The alkyl groups may be selected from methyl, ethyl, n-propyl, iso-propyl, n-butyl, or tert-butyl.

In formula (II) the substituent R³ may be selected from hydrogen, methyl, ethyl, n-propyl, iso-propyl, n-butyl, or tert-butyl, aryl or benzyl.

Starting materials according to formula (I) are commercially available or may be synthesised by methods known in the art.

The process is preferably carried out at 120 to 220 °C, 140 to 200 °C, preferably 150 to 190 °C, more preferably 165 to 175 °C. It may be carried out under stirring or without stirring, preferably under stirring.

The process is carried out under pressure. It may be carried out in an autoclave under autogenous pressure, i.e. the pressure in the autoclave is generated by heating to achieve above reaction temperatures and is dependent on the volume of the autoclave. The pressure in the autoclave may be 2 to 10 bar, preferably be 2 to 7 bar, more preferably 3 to 6 bar, even more preferably 4 to 6 bar, or 4 to less than 6 bar.

The process may alternatively be carried out in a pressure reactor by applying pressure externally, for example by exposing the reaction mixture to a gas pressure. The pressure to be achieved in the pressure reactor may be the one stated above.

Since the process is carried out at a much lower pressure (below 10 bar) than processes of the prior art, simpler and less costly reactors may be used, in particular when the reaction is run below 6 bar.

The process may alternatively be carried out in continuous mode using a tubular reactor, wherein aforesaid pressure ranges may be applied on a solution by a pump effecting compression of a solution.

Neither an aqueous ammonia solution is mixed with the starting materials and other reagents (if any), nor gaseous ammonia (NH₃) is fed into the reaction solution.

The substitution of the hydroxy group by an amino group is achieved by the addition of a salt comprising an ammonium cation or alkyl-substituted ammonium cation.

Also, the said salt may have a solubility of 0.5 mol/liter to 25 mol/liter, preferably at least 1 mol/liter to 25 mol/liter, wherein the solubility is measured at room temperature.

The aforesaid salt comprises an anion that may have an equilibrium constant for a base on a logarithmic scale (pK_{b}) of 20 or lower, preferably 16 or lower, more preferably 11 or lower, wherein the lower limit is 1 or higher.

The salt may comprise a cation selected from ammonium (NH₄⁺) or a mono-alkyl-substituted ammonium (NR³H₃⁺), wherein R³ is as defined above (with the exclusion of hydrogen).

The salt may comprise an anion selected from the group consisting of fluoride, chloride, bromide, iodide, sulfate, hydrogensulfate, sulfite, phosphate tribasic, phosphate dibasic, phosphate monobasic, hydrogencarbonate, carboxylate such as acetate, propionate, butyrate, citrate, oxalate; formate, mesylate, triflate and tosylate.

The aforesaid salt may be added in 2 to 10, preferably 3 to 7, more preferably 4 to 6 molar equivalents based on formula (I), wherein the molar equivalents are calculated with respect to the ammonium cation of the salt.

In a particular embodiment of the process, the aforesaid salt used in the process may be formed in situ by an addition of a corresponding base of the cations described above and a corresponding acid of the anions described above in required respective molar ratios. The corresponding bases and corresponding acids may be added in the molar equivalents based on formula (I) as described in the paragraphs above.

The process may be carried out under addition of an acid so that the reaction solution attains a pH of 5 to 8, preferably 6 to 7. The acid may be a mineral acid, a carboxylic acid or a sulfonic acid. The acid may be selected from the group consisting of hydrofluoric acid, hydrochloric acid, hydrobromic acid, hydroiodic acid, sulfuric acid (H₂SO₄), sulfurous acid (H₂SO₃), nitric acid (HNO₃), phosphoric acid (H₃PO₄), acetic acid, glacial acetic acid, citric acid, formic acid, lactic acid, oxalic acid, malic acid, tartaric acid, butyric acid, methanesulfonic acid, triflic acid, and toluene sulfonic acid. Preferred acids are acetic acid or glacial acetic acid. The addition of an acid has the advantage of reducing the pressure built up in a reactor.

The process may be carried out under addition of an inert salt at a concentration of 0.1 to 0.4, preferably 0.15 to 0.3 molar equivalents based on formula (I). The inert salt may be a salt comprising an alkali metal cation, earth alkaline metal cation, quaternary ammonium cation or a transition metal cation and an anion selected from the group consisting of fluoride, chloride, bromide, iodide, sulfate, sulfite, phosphate tribasic, phosphate dibasic, phosphate monobasic, acetate, propionate, butyrate, citrate, formate, mesylate, triflate and tosylate, wherein the inert salt is water soluble, meaning it may have a solubility of 0.5 mol/liter of up to 25 mol/liter, preferably at least 1 mol/liter to 25 mol/liter, wherein the solubility is measured at room temperature. Preferred inert salts are sodium sulfate, potassium sulfate, or sodium tosylate. As inert salts such salts are used which do not show any catalytic activity.

The addition of the inert salt has the advantage of reducing the pressure built up in a reactor.

In particular, the present disclosure provides a process for the production of 4-amino-5-methyl-(1H)-pyridin-2-one of formula (IV), characterized in that 4-hydroxy-5-methyl-(1H)-pyridin-2-one of formula (III) 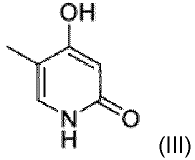 is reacted under pressure with a salt comprising an ammonium cation and in the absence of ammonia (NH₃) to afford 4-amino-5-methyl-(1H)-pyridin-2-one of formula (IV)

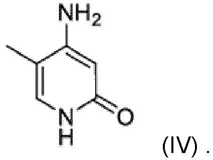

The starting material, 4-hydroxy-5-methyl-(1H)-pyridin-2-one (formula (III)), is commercially available or may be synthesised according to the method described in the article in Synthesis, 1984, volume 9, pages 765 to 766.

The process for the production of 4-amino-5-methyl-(1H)-pyridin-2-one of formula (IV) may be carried out under the conditions and using the reagents described above.

The process may be carried out at the temperatures given above. A preferred range may be 150 to 190 °C, more preferably 165 to 175 °C.

The process may be carried out under the pressure regimes given above. A preferred pressure regime may be 2 to 7 bar, more preferably 3 to 6 bar, even more preferably 4 to 6 bar, or 4 to less than 6 bar. These values refer to a process carried out in an autoclave and a process, respectively, wherein pressure is applied externally.

The process for the production of formula (IV) is carried out under addition of a salt comprising an ammonium cation (NH₄⁺). The salt may have a solubility as described above, and comprise an anion having a pK_{b} as described above.

The anion may be selected from the group consisting of fluoride, chloride, bromide, iodide, sulfate, hydrogensulfate, sulfite phosphate tribasic, phosphate dibasic, phosphate monobasic, hydrogencarbonate, carboxylate such as acetate, propionate, butyrate, citrate, oxalate; formate, mesylate, triflate and tosylate.

Preferably, the salt may comprise an ammonium cation and an anion selected from the group consisting of fluoride, sulfite, phosphate tribasic, phosphate dibasic, hydrogencarbonate, acetate, citrate, oxalate, formate, mesylate. Even more preferably, the salt may be selected from the group consisting of ammonium acetate, ammonium tribasic phosphate and ammonium dibasic phosphate. The salt may be ammonium acetate.

The aforesaid salt may be added in 2 to 10, preferably 3 to 7, more preferably 4 to 6 molar equivalents based on formula (III), wherein the molar equivalents are calculated with respect to the ammonium cation of the salt.

The process may be carried out under addition of an acid so that the reaction solution attains a pH of 5 to 8, preferably 6 to 7. The acids that may be used are the acids described above.

The process may be carried out under addition of an inert salt at a concentration of 0.1 to 0.4, preferably 0.15 to 0.3 molar equivalents based on formula (III). The salts useable are those described above.

In another embodiment, the processes as disclosed in the above paragraphs are carried out by additionally adding a Lewis acid catalyst. A salt of a metal such as magnesium, calcium, aluminium, boron, silicon, zinc, iron, copper, silver, titanium and zirconium may be added to the reaction. The counter ion of the salt may be an inorganic anion such as halides, preferably fluoride, chloride, bromide and iodide, as well as sulfate, sulfite, phosphates, preferably phosphate tribasic, or organic anions such as carboxylates, e.g. acetate, formate, citrate, and sulfonates, preferably mesylate, tosylate, triflate. As Lewis acid catalyst zinc acetate, aluminium lactate, magnesium chloride, calcium chloride, or iron(III) chloride may be used. The Lewis acid catalyst may be added in the form of a powder. The proportion of the catalyst may be 5 to 20 mol %, or 5 to 15 mol %, or 7 to 13 mol %, or 8 to 12 mol % with regard to the starting material (1H)-pyridine-2-one. The proportions of the salt comprising an ammonium cation may be as described above. In an example of the process, a (1H)-pyridine-2-one is reacted with ammonium acetate and as Lewis acid catalyst aluminium lactate, zinc acetate, magnesium chloride, calcium chloride, or iron(III) chloride.

The temperatures applied in this embodiment may be as described above. However, they can be as low as 130 to 170 °C, 140 to 160 °C, or 145 to 155 °C, preferably 150 °C. The pressure of the process may be as described above.

The advantage of the addition of a Lewis acid catalyst is that the reaction proceeds at lower temperatures and lower pressures and gives high yields.

In general, the processes described above may be carried out using only water as solvent. This has the advantage that work-up procedures are simple. In an embodiment, the product crystallises from the reaction solution in such high purity that even recrystallisation is unnecessary.

Alternatively, a solvent including n-alkanols, branched alkanols, polyols and polyethers may be added. The solvent may be added at 10 to 50 %(v/v). For example, the solvent may be added as part of a solution comprising the (1H)-pyridine-2-one starting material (III).

The processes described above may be carried out for 15 to 48 hours, preferably 20 to 30 hours. However, they can be run up to two weeks without detrimental effect on the yield.

After completion of the reaction the crude reaction mixture may be subjected to standard work-up procedures. For example, the crude reaction mixture may be cooled to induce crystallisation, after which the product is isolated by filtration. In an embodiment, the final product crystallises from the reaction solution in such high purity that recrystallisation is unnecessary. Further time and resource-consuming purification steps, such as chromatographic purification, are not necessary. Products are obtained in high purity (> 99% as per HPLC measurements). Yields of 50 to 80% are obtained.

The present invention will be better understood with reference to the following examples and figures. These examples are intended to be representative of specific embodiments of the invention, and are not intended as limiting the scope of the invention.

### Examples

### Materials & Methods

The starting material, 4-hydroxy-5-methyl-(1H)-pyridin-2-one (formula (I)), is commercially available or synthesised according to the method described in Synthesis, 1984, volume 9, pages 765 to 766. The remaining reagents are commercially available.

The autoclave used was a Büchi standard lab autoclave having a volume of 100 ml.

HPLC analysis was carried out using an alkyl reversed-phase bonded phase of the type Zorbax SB-Aq (Agilent) as stationary phase, kept at 20 degrees Celsius. As mobile phase ammonium acetate buffer at a pH of 5.7 was used, which was injected during initial and final phases. Intermittently 40 to 60 vol.% acetonitrile were added to the ammonium acetate buffer at a pH of 5.7.

Yields in the examples below indicate yields after work-up and drying.

### Example 1

12.5 g (0.1 mol) 4-hydroxy-5-methyl-2(1*H*)-pyridin-2-one (compound III) was added to a solution of 39.9 g (0.5 mol) ammonium acetate dissolved in 13 ml water. Within 4 hours the reaction mixture was heated to 170°C in a pressure reactor under autogenous pressure (5.7 bars). After 170°C were reached, the mixture was kept at that temperature for 48 hours and then allowed to cool to 60°C. The mixture is diluted with ammonia water (25%) and cooled to 0°C. The precipitated crystalline solid was filtered, washed with ethanol and dried under vacuum to give 8.9 g (70 % yield) 4-amino-5-methyl-2(1*H*)-pyridine-2-on (compound IV): purity by HPLC: 99.8%, IR spectrum compliant.

Under the same reaction conditions, salts given in the table below were reacted yielding the following results:
(170 °C, autogenous pressure built up to ca. 5.7 bars, 1 eq. reactant)

| Ammonium (NH₄⁺) salt | Salt [eq.] | Conversion [%] | Selectivity [%] | pKb | Solubility [mol/l] at 20°C |
|---|---|---|---|---|---|
| Fluoride | 5.00 | 79.70 | 92.70 | 10.68 | 22.55 |
| Formiate | 5.00 | 88.10 | 81.30 | 10.23 | 10.51 |
| Sulfite | 5.00 | 90.00 | 11.11 | 6.80 | 2.79 |
| Phosphate | 1.67 | 93.42 | 83.67 | 1.64 | 3.89 |
| Hydrogenphosphate | 5.00 | 94.90 | 97.70 | 6.80 | 1.00 |
| Hydrogencarbonate | 5.00 | 95.80 | 99.00 | 7.48 | 2.78 |
| Citrate | 1.00 | 94.96 | 78.82 | 7.60 | 4.11 |
| Oxalate | 5.00 | 92.3 | 93.9 | 9.81 | 0.36 |
| Acetate | 5.00 | 95.35 | 97.28 | 9.25 | 19.22 |

(Conversion determined by HPLC under the above conditions.)

### Example 2 (adding an acid to reduce the pH)

Glacial acetic acid was added to a solution of 39.9 g (0.5 mol) ammonium acetate dissolved in 13 ml water until a pH of 7.0 is reached. 12.5 g (0.1 mol) 4-hydroxy-5-methyl-2(1*H*)-pyridin-2-one (compound III) were added to the neutral ammonium acetate solution. Within 4 hours the reaction mixture was heated to 170°C in a pressure reactor under autogenous pressure (4.7 bars). After 170°C were reached, the mixture was kept at that temperature for 48 hours and then allowed to cool to 60°C. The mixture is diluted with ammonia water (25%) and cooled to 0°C. The precipitated crystalline solid was filtered, washed with ethanol and dried under vacuum to give 8.6 g (69 % yield) 4-amino-5-methyl-2(1*H*)-pyridine-2-on (compound IV): purity by HPLC: 99.6%, IR spectrum compliant.

### Example 3 (adding sodium sulphate as inert salt)

12.5 g (100 mmol) 4-hydroxy-5-methyl-2(1*H*)-pyridin-2-one (compound III) and 2.0 g sodium sulphate (14.1 mmol) were added to a solution of 39.9 g (0.5 mol) ammonium acetate dissolved in 13 ml water. Within 4 hours the reaction mixture was heated to 170°C in a pressure reactor under autogenous pressure (5.3 bars). After 170°C were reached, the mixture was kept at that temperature for 48 hours and then allowed to cool to 60°C. The mixture is diluted with ammonia water (25%) and cooled to 0°C. The precipitated crystalline solid was filtered, washed with ethanol and dried under vacuum to give 9.2 g (72 % yield) 4-amino-5-methyl-2(1*H*)-pyridine-2-on (compound IV): purity by HPLC: 99.6%, IR spectrum compliant.

### Example 4 (process at at 190 °C instead of 170 °C)

7.0 g (0.06 mol) 4-hydroxy-5-methyl-2(1*H*)-pyridin-2-one (compound III) was added to a solution of 27.9 g (0.36 mol) ammonium acetate dissolved in 8.4 ml water. Within 13 hours the reaction mixture was heated to 190°C in a pressure reactor under autogenous pressure (8 bars). After 190°C were reached, the mixture was kept at that temperature for 48 hours and then allowed to cool to 20°C. The mixture is diluted with ammonia water (25%) and cooled to 0°C. The precipitated crystalline solid was filtered, washed with ethanol and dried under vacuum to give 5.5 g (79 % yield) 4-amino-5-methyl-2(1*H*)-pyridine-2-on (compound IV): purity by HPLC: 99.7%, IR spectrum compliant.

### Example 5 (addition of a Lewis acid)

0.33 g (2.65 mmol) 4-hydroxy-5-methyl-2(1*H*)-pyridin-2-one (compound III) was added to 1.02 g (13.25 mmol) ammonium acetate and 0.04 g (0.27 mmol) iron(III) chloride in 0.38 g (21.2 mmol) water. The reaction mixture was heated to 170°C in a pressure reactor under autogenous pressure. After 170°C were reached, the mixture was kept at that temperature for 72 hours and then allowed to cool to 20°C. The conversion was monitored by HPLC to be 94 %.

### Example 6 (addition of a Lewis acid double salt)

0.33 g (2.65 mmol) 4-hydroxy-5-methyl-2(1*H*)-pyridin-2-one (compound III) was added to 3.51 g (13.25 mmol) ferric ammonium citrate in 0.38 g (21.2 mmol) water. The reaction mixture was heated to 170°C in a pressure reactor under autogenous pressure. After 170°C were reached, the mixture was kept at that temperature for 72 hours and then allowed to cool to 20°C. The conversion was monitored by HPLC to be 87 %.

### Example 7 (Synthesis of 4-(N-methylamino)-5-methyl-1H-pyridine-2-one)

0.33 g (2.65 mmol) 4-hydroxy-5-methyl-2(1*H*)-pyridin-2-one (compound III) was added to 1.21 g (13.25 mmol) methylammonium acetate in 0.38 g (21.2 mmol) water. The reaction mixture was heated to 170°C in a pressure reactor under autogenous pressure. After 170°C were reached, the mixture was kept at that temperature for 72 hours and then allowed to cool to 20°C. The conversion was monitored by HPLC to be 87 %. Identity was confirmed by ¹H-NMR (DMSO-d6): δ 1.83 (3H, s), 2.65 (3H, s), 5.05 (1H, s), 6.84 (1H, s).

### Embodiment section:

1. Process for the production of a 4-amino-(1H)-pyridin-2-one of formula (II) 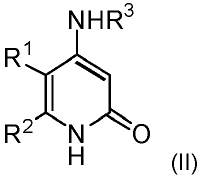 characterised in that a 4-hydroxy-(1H)-pyridin-2-one of formula (I) 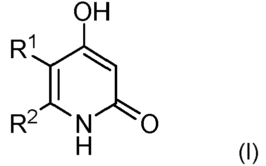
   is reacted under pressure with a salt comprising an ammonium cation and in the absence of ammonia,
   wherein R¹ and R² are independently selected from linear or branched alkyl groups, aryl, benzyl, or hydrogen, and
   R³ is selected from hydrogen, methyl, ethyl, n-propyl, iso-propyl, n-butyl, or tert-butyl, aryl, or benzyl.
2. Process according to claim 1, characterized in that the reaction temperature is 120 to 220 °C.
3. Process according to claim 1 or claim 2, characterized in that the reaction temperature is 150 to 190 °C.
4. Process according to any of claims 1 to 3, characterized in that the reaction temperature is 165 to 175 °C.
5. Process according to any of claims 1 to 4, characterized in that the salt comprises an anion having an equilibrium constant for a base on a logarithmic scale (pK_{b}) of 20 or lower, preferably 16 or lower, and most preferably 11 or lower, wherein the lower limit is 1 or higher.
6. Process according to claim 5, characterized in that the salt has a solubility of 0.5 mol/liter of up to 25 mol / liter, preferably at least 1 mol / liter to 25 mol / liter, wherein the solubility is measured at room temperature.
7. Process according to any of claims 1 to 6, characterized in that the salt comprises a cation comprising ammonium or (NH₃R³)⁺
   and an anion selected from the group consisting of fluoride, chloride, bromide, iodide, sulfate, hydrogensulfate, sulfite, phosphate tribasic, phosphate dibasic, phosphate monobasic, hydrogencarbonate, acetate, citrate, propionate, butyrate, oxalate, formate, mesylate, triflate and tosylate.
8. Process according to claim 7, characterized in that the salt comprises an ammonium cation or (NH₃R³)⁺ and an anion selected from the group consisting of fluoride, sulfite, phosphate tribasic, phosphate dibasic, hydrogencarbonate, acetate, citrate, oxalate, formate, mesylate.
9. Process according to claim 8, characterized in that the salt comprises an ammonium cation, or an N-methyl-ammonium cation, or an N-ethyl-ammonium cation and an anion selected from the group consisting of fluoride, phosphate tribasic, phosphate dibasic, hydrogen carbonate, acetate, citrate, oxalate, formate.
10. Process according to any of claims 1 to 9, characterized in that the salt is selected from the group consisting of ammonium acetate, ammonium tribasic phosphate and ammonium dibasic phosphate.
11. Process according to any of claims 1 to 10, characterized in that the salt is ammonium acetate.
12. Process according to any of claims 1 to 11, characterized in that the salt is added in 2 to 10, preferably 3 to 7, more preferably 4 to 6 molar equivalents based on formula (I), wherein the molar equivalents are calculated with respect to the ammonium cation of a salt.
13. Process according to any of claims 1 to 12, characterized in that R¹ and R² are independently selected from the group consisting of methyl, ethyl, n-propyl, iso-propyl, n-butyl, tert-butyl, benzyl, aryl, and hydrogen.
14. Process according to any of claims 1 to 13, characterized in that the reactant is 4-hydroxy-5-methyl-(1H)-pyridin-2-one, which is reacted to give 4-amino-5-methyl-(1H)-pyridin-2-one.
15. Process according to any of claims 1 to 14, characterized in that an acid is added so that the reaction solution attains a pH of 5 to 8, preferably 6 to 7.
16. Process according to claim 15, characterized in that the acid is a mineral acid, a carboxylic acid or a sulfonic acid.
17. Process according to claim 16, characterized in that the acid is selected from the group consisting of hydrofluoric acid, hydrochloric acid, hydrobromic acid, hydroiodic acid, sulfuric acid (H₂SO₄), sulfurous acid (H₂SO₃), nitric acid (HNO₃), phosphoric acid (H₃PO₄), acetic acid, glacial acetic acid, citric acid, formic acid, lactic acid, oxalic acid, malic acid, tartaric acid, butyric acid, methanesulfonic acid, triflic acid, and toluene sulfonic acid.
18. Process according to any of claims 1 to 17, characterized in that an inert salt is added at a concentration of 0.1 to 0.4, preferably 0.15 to 0.3 mol based on formula (I).
19. Process according to claim 18, characterized in that the inert salt comprises salts comprising an alkali metal cation, earth alkaline metal cation, quaternary ammonium cation or a transition metal cation and an anion selected from the group consisting of fluoride, chloride, bromide, iodide, sulfate, sulfite phosphate tribasic, phosphate dibasic, phosphate mono basic, acetate, proprionate, butyrate, citrate, formate, mesylate, triflate and tosylate, wherein the inert salt is water soluble.
20. Process according to any of claims 1 to 19, characterized in that pressure is built up in an autoclave.
21. Process according to any of claims 1 to 19, characterized in that the reaction is carried out in a pressure reactor by applying an external pressure of 2 to 10 bar, preferably 2 to 7 bar, more preferably 3 to 6 bar, even more preferably 4 to 6 bar.
22. Process according to any of claims 1 to 21, characterized in that the reaction is carried out in the presence of a Lewis acid catalyst.
23. Process according to any of claims 1 to 22, characterized in that the reaction is carried out in the presence of 5 to 20 mol %, or 5 to 15 mol %, or 7 to 13 mol %, or 8 to mol 12 % with regard to formula (I) of a Lewis acid catalyst.
24. Process according to claims 22 or 23, characterized in that the Lewis acid catalyst is selected from an inorganic or organic salt of magnesium, calcium, aluminium, boron, silicon, zinc, iron, copper, silver, titanium and zirconium.
25. Process according to any of claims 22 to 24, characterized that ammonium acetate is reacted with aluminium lactate, zinc acetate, magnesium chloride, iron(lll) chloride, or calcium chloride.
26. Process according to any of claims 22 to 25, characterized in that it is carried out at 130 to 170 °C, 140 to 160 °C, or 145 to 155 °C, preferably 150 °C.
27. Process according to any of claims 1 to 26, characterized in that water is used as an only solvent in the reaction.
28. Process according to any of claims 1 to 27, characterized in that a solvent is added selected from the group including n-alkanols, branched alkanols, polyols and polyethers.
29. Process according to any of claims 1 to 28, characterized in that the reaction is carried out for 15 to 48 hours, preferably 20 to 30 hours.

## Claims

1. Process for the production of a 4-amino-(1H)-pyridin-2-one of formula (II) 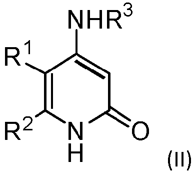 **characterised in that** a 4-hydroxy-(1H)-pyridin-2-one of formula (I) 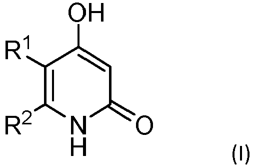
is reacted under pressure with a salt comprising an ammonium cation and in the absence of ammonia,
wherein R¹ and R² are independently selected from linear or branched alkyl groups, aryl, benzyl, or hydrogen, and
R³ is selected from hydrogen, methyl, ethyl, n-propyl, iso-propyl, n-butyl, or tert-butyl, aryl or benzyl.

2. Process according to claim 1, **characterized in that** the reaction temperature is 120 to 220 °C.

3. Process according to claim 1 or 2, **characterized in that** the salt comprises an anion having an equilibrium constant for a base on a logarithmic scale (pK_{b}) of 20 or lower, preferably 16 or lower, and most preferably 11 or lower, wherein the lower limit is 1 or higher.

4. Process according to claim 3, **characterized in that** the salt has a solubility of 0.5 mol/ liter of up to 25 mol / liter, preferably at least 1 mol / liter to 25 mol / liter, wherein the solubility is measured at room temperature.

5. Process according to any of claims 1 to 4, **characterized in that** the salt comprises a cation comprising ammonium or (NH₃R³)⁺
and an anion selected from the group consisting of fluoride, chloride, bromide, iodide, sulfate, hydrogensulfate, sulfite, phosphate tribasic, phosphate dibasic, phosphate monobasic, hydrogencarbonate, acetate, propionate, butyrate, citrate, oxalate, formate, mesylate, triflate and tosylate.

6. Process according to any of claims 1 to 5, **characterized in that** the salt is selected from the group consisting of ammonium acetate, ammonium tribasic phosphate and ammonium dibasic phosphate.

7. Process according to any of claims 1 to 6, **characterized in that** the salt is added in 2 to 10 molar equivalents based on formula (I), wherein the molar equivalents are calculated with respect to the ammonium cation of a salt.

8. Process according to any of claims 1 to 7, **characterized in that** R¹ and R² are independently selected from the group consisting of methyl, ethyl, n-propyl, iso-propyl, n-butyl, tert-butyl, aryl, benzyl, and hydrogen.

9. Process according to any of claims 1 to 8, **characterized in that** the reactant is 4-hydroxy-5-methyl-(1H)-pyridin-2-one, which is reacted to give 4-amino-5-methyl-(1H)-pyridin-2-one.

10. Process according to any of claims 1 to 9, **characterized in that** an acid is added so that the reaction solution attains a pH of 5 to 8, preferably 6 to 7.

11. Process according to claim 10, **characterized in that** the acid is a mineral acid, a carboxylic acid or a sulfonic acid.

12. Process according to any of claims 1 to 11, **characterized in that** an inert salt is added at a concentration of 0.1 to 0.4 mol based on formula (I).

13. Process according to claim 12, **characterized in that** the inert salt comprises salts comprising an alkali metal cation, earth alkaline metal cation, quaternary ammonium cation or a transition metal cation and an anion selected from the group consisting of fluoride, chloride, bromide, iodide, sulfate, hydrogensulfate, sulfite, phosphate tribasic, phosphate dibasic, phosphate monobasic, acetate, propionate, butyrate, citrate, formate, mesylate, triflate and tosylate, wherein the inert salt is water soluble.

14. Process according to any of claims 1 to 13, **characterized in that** the reaction is carried out in the presence of a Lewis acid catalyst in 5 to 20 mol % with regard to formula (I).

15. Process according to claim 14, **characterized in that** it is carried out at 130 to 170 °C.
